# EUROPEAN PATENT APPLICATION

(11) **EP 2 308 488 A1**
(43) Date of publication of application: **13.04.2011**
(21) Application number: 09290768.2
(22) Date of filing: 09.10.2009
(51) Int. Cl.: A61K 31/343, A61P 9/06

(54) **Use of celivarone for the preparation of a medicament for use in the prevention of implantable cardioverter defibrillator interventions or death**

(71) Applicant: Sanofi-Aventis, 75013 Paris (FR)
(72) Inventor: Gaudin, Christophe, 75013 Paris (FR); Radzik, Davide, 75013 Paris (FR)
(74) Representative: Romanowski, Caroline

(57) **Abstract**

The invention relates to the use of celivarone, or a pharmaceutically acceptable salt thereof, for the preparation of a medicament for use in the prevention of Implantable Cardioverter Defibrillator interventions or death.

## Description

The present invention relates to the use of celivarone or a pharmaceutically acceptable salt thereof for the preparation of a medicament for use in the prevention of Implantable Cardioverter Defibrillator interventions or of death.

The management of patients with symptomatic and/or potentially life-threatening ventricular arrhythmias is a significant clinical problem. As with atrial tachyarrhythmia, therapy can be divided into acute treatment, for the rapid restoration of sinus rhythm from ventricular fibrillation (VF) or ventricular tachycardia (VT), and chronic treatment for the prevention of further episodes of VT or VF in patients who have already experienced at least one such arrhythmia. In the acute treatment of VT and VF, the treatment of choice is usually directy current shock delivered either externally or by an Implantable Cardioverter Defibrillator (ICD). About 20-50% of patients with an ICD are additionally treated with antiarrhythmic drugs.

Celivarone is an antiarrhythmic benzofuran derivative currently in clinical development for the treatment of ventricular arrhythmia. Celivarone, or 2-butyl-3-[4-[3-(dibutylamino)propyl]benzoyl]-1-benzofuran-5-carboxylic acid isopropyl ester fumarate, and other pharmaceutically acceptable salts thereof are described in the international patent application WO 02/16339.

The applicant has obtained clinical data suggesting that celivarone could be effective for the prevention of ventricular arrhythmia triggered interventions in ICD patients, these data warranting further investigation (ICARIOS study, see J. Am. Coll. Cardiol. 2008, 51(10, Suppl. A), abstract 1001-94).

The applicant demonstrates herein that celivarone is effective for the prevention of Implantable Cardioverter Defibrillator (ICD) interventions or death, in a clinical trial involving a larger population and using as a calibrator the drug amiodarone, an antiarrhythmic benzofuran derivative commercialized since more than forty years.

The subject-matter of the invention is the use of a compound chosen from celivarone and pharmaceutically acceptable salts thereof for the preparation of a medicament for use in the prevention of Implantable Cardioverter Defibrillator (ICD) interventions or death, in particular sudden death.

According to the present invention, "ICD interventions" designates ventricular tachycardia or ventricular fibrillation triggered ICD interventions, comprising ICD shocks and ATPs (antitachycardia pacings).

According to the instant invention, celivarone or a pharmaceutically acceptable salt thereof is advantageously administered by the oral route.

In an embodiment of the invention, celivarone or a pharmaceutically acceptable salt thereof is administered at a daily dose comprised between 50 and 300 mg, advantageously between 100 and 300 mg.

In another embodiment, celivarone or a pharmaceutically acceptable salt thereof is taken with a meal.

In another embodiment of the invention, celivarone or a pharmaceutically acceptable salt thereof is administered to patients having an Implantable Cardioverter Defibrillator and having a Left Ventricular Ejection Fraction of 40% or less.

According to the invention, the compound administered in order to prevent Implantable Cardioverter Defibrillator (ICD) interventions or death is 2-butyl-3-[4-[3-(dibutylamino)propyl]benzoyl]-1-benzofuran-5-carboxylic acid isopropyl ester fumarate. Said compound advantageously enables to prevent Implantable Cardioverter Defibrillator interventions. Said compound also advantageously enables to prevent death.

The invention will be more clearly understood by reference to the following example of the invention, outlining the clinical study ALPHEE sponsored by sanofi-aventis.

The following abbreviations shall be used:
ACC/AHA : American College of Cardiology / American Heart Association
ALT: Alanine Aminotransferase
AST: Aspartate Aminotransferase
ATP: Antitachycardia Pacing
ICD: Implantable Cardioverter Defibrillator
LVEF : Left Ventricular Ejection Fraction
VT: Ventricular Tachycardia
VF : Ventricular Fibrillation

### - Study objective

The primary objective of the study is to assess the efficacy of celivarone for the prevention of ICD interventions or death.

### - Study design

This is a dose-finding multicenter, multinational, randomized, double blind, placebo controlled, parallel arm study including a positive control calibrator arm. Patients are randomized to one of three doses of celivarone or placebo or to the amiodarone calibrator arm.

To be eligible, patients must have an implantable cardioverter defibrillator (see below for detailed inclusion criteria).

The efficacy of celivarone is based on data regarding appropriate ICD intervention obtained by regular ICD interrogation, performed as described below, as well as on data regarding death of the randomized patients, in particular sudden death.

The study includes a one week screening period, followed by a treatment period scheduled for a minimum duration of 6 months for the last patient recruited and going from the first day of treatment to the End of Treatment visit to be done 10-15 days prior to the scheduled study end date.

### - Patients

Eligible patients are ICD patients with a LVEF of 40% or less, and one of the following criteria:
- at least one ICD therapy for VT or VF in the previous month
- or ICD implantation in the previous month for documented VT/VF.
   Concerning the exclusion criteria, a patient is not included if one or more of the following criteria is present :
   Patients of either sex aged below 21 years (or the age of legal consent of the country).

Women of childbearing potential without adequate birth control or pregnant or breastfeeding women.

Patients with known ICD lead problem (lead dislodgement).

ICD without the following characteristics :
- data logging function with cumulative counting of device intervention (shocks and antitachycardia pacing [ATP]),
- electrogram storage capabilities,
- ventricular demand pacing.

Recent unstable angina pectoris or myocardial infarction (< 4 weeks).

History of torsades de pointes.

Genetic channelopathies including congenital long QT syndrome.

Wolff-Parkinson-White syndrome.

Patients in unstable hemodynamic condition such as acute pulmonary edema within 12 hours prior to start of study medication; cardiogenic shock; treatment with intravenous pressor agents; patients on respirator; congestive heart failure of stage New York Heart Association (NYHA) IV within the last 4 weeks; uncorrected, hemodynamically significant primary obstructive valvular disease; hemodynamically significant obstructive cardiomyopathy; a cardiac operation or revascularization procedure within 4 weeks preceding randomization.

Incessant sustained VT/VF (VT/VF that recurs promptly despite termination attempts) during the three days preceding randomization.

Patients with inappropriate (not triggered by VT nor VF) shocks during the month preceding randomization.

Clinically relevant haematologic, hepatobiliary (ALT, AST > 3 times the upper limit of normal at randomization), gastro-intestinal, renal (serum creatinine > 221 µmol/l (2.5 mg/dl) at randomization), pulmonary, endocrinologic or psychiatric disease.

Patients treated with oral amiodarone (more than 20 tablets during the 2 months preceding randomization).

### - Treatments

The investigational products are celivarone (capsules of 50 or 100 mg) or amiodarone (capsules of 200 mg) and their matching placebo in capsules forms.

They are administered by the oral route, with a meal. Indeed, given a significant food effect (drug bioavailability is greatly reduced if taken fasting) it is essential that the study drug is taken with food, such as at the end of a main meal.

The daily doses are 50, 100 or 300 mg for celivarone and 200 mg for amiodarone (with a loading dose of 600 mg daily for 10 days).

### - Assessment of investigational product

VTNF triggered ICD intervention (comprising ICD shocks and ATPs) and death are recorded in the randomized patients.

Patients are instructed to call the investigator if they receive a shock or in case of syncope or dizziness, so that they can be seen as soon as possible, within a week in order to have the electrograms stored by the ICD analyzed. In case a patient experiences cluster shocks or electrical instability (>3 interventions/day), the ICD should be interrogated within one day.

ICD interrogation is performed by a trained electrophysiologist familiar with ACC/AHA guidelines for implantation of cardiac pacemakers and arrhythmia devices, at regular intervals planned during the study duration. Some patients might present a high number of ICD interventions as compared to the storage capability of his device ; the investigator carefully evaluates the need for more frequent interrogations than the planned ones, in order not to lose ICD intervention information because of device memory saturation.

In case of death of a patient during the study, the investigator does his best to perform post-mortem interrogation of the device.

## Claims

1. Use of a compound chosen from celivarone and pharmaceutically acceptable salts thereof for the preparation of a medicament for use in the prevention of Implantable Cardioverter Defibrillator interventions or death.

2. The use according to claim 1, wherein said compound is administered by the oral route.

3. The use according to claim 1 or claim 2, wherein said compound is administered at a daily dose comprised between 50 and 300 mg.

4. The use according to claim 3, wherein said compound is administered at a daily dose of 100 to 300 mg.

5. The use according to any of claims 1 to 4, wherein said compound is taken with a meal.

6. The use according to any of claims 1 to 5, wherein said medicament is administered to patients having an Implantable Cardioverter Defibrillator and having a Left Ventricular Ejection Fraction of 40% or less.

7. The use according to any of claims 1 to 6, wherein said compound is 2-butyl-3-[4-[3-(dibutylamino)propyl]benzoyl]-1-benzofuran-5-carboxylic acid isopropyl ester fumarate.

8. The use according to any of claims 1 to 7, for the prevention of Implantable Cardioverter Defibrillator interventions.

9. The use according to any of claims 1 to 8, for the prevention of ventricular tachycardia or ventricular fibrillation triggered ICD interventions, comprising ICD shocks and antitachycardia pacings.

10. The use according to any of claims 1 to 7, for the prevention of death.

11. The use according to any of claims 1 to 7 or 10, for the prevention of sudden death.
